(19) **Europäisches Patentamt**

**European Patent Office**

**Office européen des brevets**

(11) Veröffentlichungsnummer: **0 419 660 A1**

(12) # EUROPÄISCHE PATENTANMELDUNG
## veröffentlicht nach Art. 158 Abs. 3 EPÜ

㉑ Anmeldenummer: **89909894.1**

㉒ Anmeldetag: **18.04.89**

㉘ Internationale Anmeldenummer:
**PCT/SU89/00102**

㊇ Internationale Veröffentlichungsnummer:
**WO 90/12542 (01.11.90 90/25)**

㉕ Int. Cl.⁵: **A61B 17/11**

㊸ Veröffentlichungstag der Anmeldung:
**03.04.91 Patentblatt 91/14**

㊟ Benannte Vertragsstaaten:
**BE CH DE FR GB IT LI LU NL SE**

㉛ Anmelder: **KANSHIN, Nikolai Nikolaevich**
**ul. M.Filevskaya, 68-10**
**Moscow, 121433(SU)**

Anmelder: **LIPATOV, Viktor Alexeevich**
**ul. Profsojuznaya, 91-3-27**
**Moscow, 117279(SU)**

㉜ Erfinder: **KANSHIN, Nikolai Nikolaevich**
**ul. M.Filevskaya, 68-10**
**Moscow, 121433(SU)**
Erfinder: **LIPATOV, Viktor Alexeevich**
**ul. Profsojuznaya, 91-3-27**
**Moscow, 117279(SU)**

㉔ Vertreter: **Finck, Dieter et al**
**Patentanwälte v. Füner, Ebbinghaus, Finck**
**Mariahilfplatz 2 & 3**
**W-8000 München 90(DE)**

㉝ **STIFT FÜR CHIRURGISCHES NÄHGERÄT.**

㉗ Stift enthält einen Abstützungskopf (2) und zusammengestellten Schaft (1), der aus zwei Schaftteilen (5 und 6) unterschiedlicher Länge besteht, an einem von denen, an dem Schaftteil (5) der Abstützungskopf (2) angeordnet ist, wobei dieser Schaftteil (5) in Bezug auf den anderen Schaftteil (6) festgelegt wird.

FIG. 1

## STIFT FÜR CHIRURGISCHES NÄHGERÄT

Technisches Gebiet

Die vorliegende Erfindung bezieht sich auf chirurgische Mittel, insbesondere betrifft sie Stifte für chirurgisches Nähgerät.

Zugrundeliegender Stand der Technik

Bereits ist bekannt ein Stift für das chirurgische Nähgerat, der als Schaft ausgeführt ist (GB, A, 2133735). An einem Ende desselben ist eine Matrize zum Zusammenbiegen der Klammern vermittels einer Mutter befestigt. An dem erwähnten Schaft werden die Enden der zusammenzunähenden Organe vermittels der Tabaksbeutelnaht festgelegt.

Bekannt ist auch ein weiterer Stift für das chirurgische Nähgerät, ausgeführt als ein Schaft, der einem Ende Abstützungskopf trägt. Um eine ringförmige Anastomose anzulegen, wird im voraus an diesem Schaft das Gewebe eines der zusammenzunähenden Organe mit Hilfe von der Tabaksbeutelnaht befestigt, (SU, A, 906540; SU, A, 906541).

Der aus dem Nähgerät herausgenommene Stift weist eine große Länge auf, wodurch die Durchführung der Operationen mit dessen Hilfe an den Abschnitten mit eingeschränktem Raum unmöglich wird. Darüber hinaus muß eine zusätzliche Tabaksbeutelnaht an dem am Stift aufgesetzten Gewebe während der Operation bei der Benutzung dieses bekannten Stiftes angelegt werden. Die aufgezählten Nachteile führen zur Herabsetzung der Asepsissicherung und zur Steigerung der Verletzbarkeit im Laufe der Operation.

Offenbarung der Erfindung

Der vorliegenden Erfindung liegt die Aufgabe zugrunde, einen Stift für das chirurgische Nähgerät zu entwickeln, der durch Veränderung dessen Länge ohne Anlegen einer Tabaksbeutelnaht an dem an diesem Stift aufgeset zten biologischen Gewebe auszukommen ermöglicht.

Die gestellte Aufgabe wird dadurch gelöst, daß im zur Anlegung von ringförmigen Anastomosen an den Organen des Verdauungskanals vorausbestimmten Stift für das chirurgische Nähgerät, der einen Schaft vorsieht, an dessen einem Ende Abstützungskopf zur Formgebung der Naht einer ringförmigen Anastomose angeordnet ist, erfindungsgemäß dieser Schaft aus zwei Schaftteilen unterschiedlicher Länge zusammengesetzt ausgeführt ist, von denen der Schaftteil mit dem Abstützungskopf eine kleinere Länge als bei dem anderen Schaftteil aufweist und in Bezug auf diesen anderen Schaftteil festgelegt werden kann.

Der Schaftteil mit dem Abstützungskopf kann außerdem unter Ermöglichung; das zusammenzunähende Organ durchzustechen, ausgeführt werden.

Um diese Möglichkeit, das zusammenzunähende Organ durchzustechen, zu erreichen, ist das freie Ende des Schaftes mit dem Abstützungskopf zweckmäßigerweise zugespitzt ausgeführt.

Möglicherweise kann der Schaft ein abnehmbares, an dem freien Ende des Schaftteils mit dem Abstützungskopf fixierendes Ansatzstück einschließen.

Die betreffende Ausführung des Stiftes für die chirurgischen Nähgeräte bietet die Möglichkeit, ohne Anlegung eines Tabaksbeutelnach an dem an dem Stift befindlichen biologischen Gewebe auszukommen, und bringt die für die Operationen an den Abschnitten im eingeschränktten Raum notwendigen Bedingungen mit sich.

Kurzbeschreibung der Erfindung

Nachstehend wird die Erfindung anhand der Beschreibung deren Ausführungsbeispiele und angelegten Zeichnungen näher erläutert, in denen es zeigt:

Fig. 1  erfindungsgemäßen Stift für das chirurgische Nähgerät;

Fig. 2  einen erfindungsgemäßen, das zugespitzte Ende aufweisenden Schaftteil des Stiftes;

Fig. 3  andere Variante der Ausführung des erfindungsgemäßen Stiftes;

Fig. 4  Stellungslage des erfindungsgemäßen Stiftes in Bezug auf das zu operierende Organ;

Fig. 5  Stellungslage des erfindungsgemäßen Stiftes in Bezug auf das zu operierende Organ nach dessen Hindurchstechen;

Fig. 6  Ausnutzungsvariante des erfindungsgemäßen Stiftes im chirurgischen Nähgerät.

Beste Ausführungsvariante der Erfindung

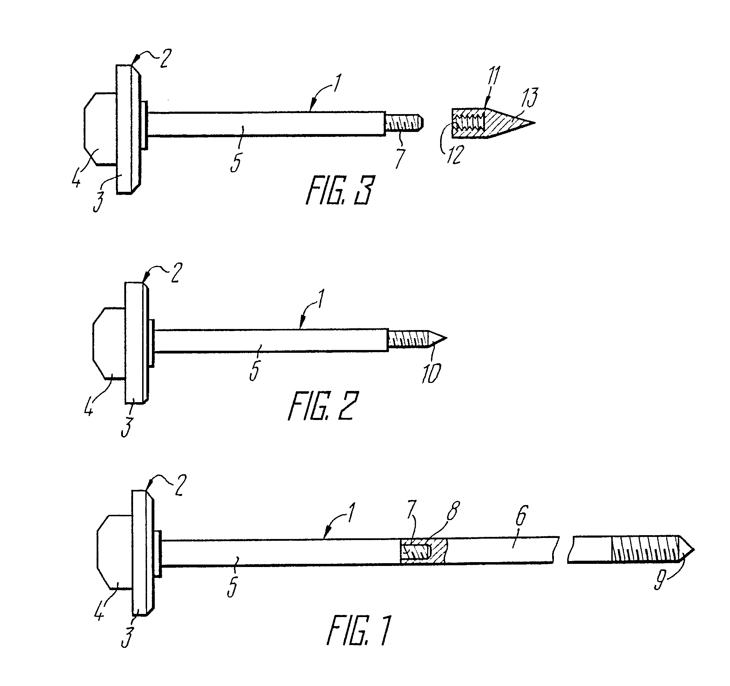

Der Stift für das chirurgische Nähgerät dient zum Anlegen von ringförmigen Anastomosen an den Organen des Verdauungskanals. Der Stift sieht einen Schaft 1 (Fig. 1), an dessen einem Ende ein Abstützungskopf 2 zur Formgebung der Nacht der ringförmigen Anastomose angeordnet ist, vor. Der Abstützungskopf 2 ist in seinem Aufbau bekannterweise ausgeführt und enthält einen Verbindungsring 3, der an dem Schaft 1 mit Hilfe der Mutter 4 festaufgesetzt ist.

Der Schaft 1 besteht aus zwei Schaftteilen 5 und 6 einer unterschiedlichen Länge, wobei der Schaftteil 6 des Schaftes 1 eine größere Länge als der Schaftteil 5 mit dem Abstützungskopf 2 aufweist, trotzdem wird der Schaftteil 6 an der Fig. 1 und den anderen Figuren zwecks Vereinfachung und Bequemlichkeit der grafischen Darstellung bedingt abgekürzt wiedergegeben.

Der Schaftteil 5 wird in Bezug auf den anderen Schaftteil 6 beispielsweise vermittels eines Gewindezapfens 7 und einer Gewindebohrung 8 festgehalten.

Der Stift ist derart ausgeführt, daß mit dessen Hilfe das Zusammennähungsorgan hindurchgestochen werden kann, wozu dessen freies Ende, insbesondere das freie Ende 9 des Schaftteils 6 zugespitzt ausgeführt ist.

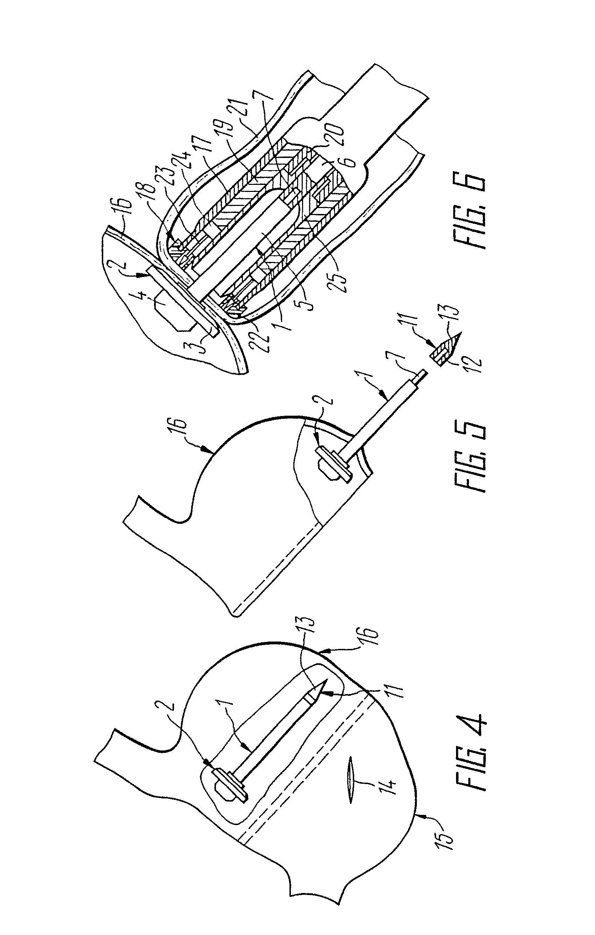

Jedoch erweis es sich als bequem, zur Durchführung der Operationen an den schwerzugänglichen Abschnitten des Körpers von Patienten oder in einem eingeschränkten Operationsfeld das freie Ende 10 (Fig. 2) des Schaftteils 5, dessen Länge im wesentlichen kleiner als diese des anderen Schaftteils 6 (Fig. 1) ist, zugespitzt auszuführen.

In Fig. 3 ist eine Ausführungsvariante des Teils 5 des Schaftes 1 wiedergegeben, der ein abnehmbares Ansatzstück 11 enthält, das mit seiner Bohrung 12 an dem Gewindezapfen 7 des freien Endes des erwähnten Teils 5 des Schaftes 1 festgelegt ist und sein anderes zugespitztes Ende 13 aufweist.

In Fig. 4 und 5 sind einige Phasen der Operation dargestellt, die unter Ausnutzung des erfindungsgemäßen Stiftes durchgeführt wird. Zunächst wird der Stift in zwei seine Teile 5 und 6 auseinandergenommen und durch einen im abzuschneidenden Teil 15 eines Organs, beispielsweise des Magens 16 ausgeführten Einschnitt 14 in die Höhle des Magens 16 eingeführt, indem er dort derart zurechtgelegt wird, daß nach dem Abschneiden des Teils 15 er innerhalb der Höhle des Magens 16 immernoch verbleibt. Dabei wird der Stift im Magen ausgerichtet und das zugespitzte Ende 13 des Ansatzstückes 11 in Richtung des Durchstechens der Wandung des Magens 16 zugekehrt.

Eine Operation mit Hilfe des Stiftes verläuft unter Ausnutzung des chirurgischen Nähgeräts wie folgt.

Nachdem die Wandung des Magens 16 vermittels des Ansatzstückes 11 hindurchgestochen wird, wird dieses Ansatzstück 11 von dem Teil 5 des Schaftes 1 mit dem Abstützungskopf 2 abgenommen, während der Schaftteil 6 (Fig. 6) an das Nähgerät, angeschlossenen in diesem festeingeklemmt wird. Das erwähnte Nähgerät schließt sein Gehäuse 17, eine Nadelvorrichtung 18, einen Stößel 19 und eine Hohlröhre 20, durch welche der Schaft 1 des Stiftes hindurchläuft, ein. Das Nähgerät wird innerhalb des Anastomosendarmes 21 untergebracht. Hiernach wird der Verbindungsring 3 an dem Abstützungskopf 2 mit dem Ring 22 der Nadelvorrichtung 18 nahgestellt und die Gewebe der zusammenzunähenden Organe, des Magens 16 und des Darmes 21 werden mit den an dem Ring 24 der Nadelvorrichtung 18 befestigten Nadeln 23 hindurchgestochen. Hiernach wird die Öffnung der Anastomose mit dem zylindrischen Messer 25 herausgeschnitten, wonach das ganze Nähgerät und der Stift ohne den Verbindungsring 3 aus der Höhle des Darmes 21 in an sich bekannter Weise herausgenommen werden.

Mit Hilfe des Nähgerätes, in dem der erfindungsgemäße Stift ausgenutzt worden ist, sind 35 Anastomosen am Magen und Dünndarm angelegt worden. Die Operationen sind erforderlich vergangen, es sind keine Komplikationen nachgewiesen.

Durch Vermeidung der Tabaksbeutelnaht an dem am Stift aufgeschobenen Gewebe machte es die Ausnutzung des erfindungsgemäßen Stiftes möglich, die Aseptikverhältnisse bei der Operation stark zu verbessern, die Gefahr der Entstehung der jeweiligen Komplikationen herabzusetzten, die sonst durch eventuelles Hinausstoßen von den durch die Tabaksbeutelnähte gebundenen Geweben aus dem Bereich der mechanischen Naht auftreten können.

Industrielle Verwertbarkeit

Die erwähnte Erfindung kann bei den Operationen an Organen des Magen-Darmkanals ihre Anwendung finden.

**Ansprüche**

1. Stift für chirurgisches Nähgerät, das zum Anlegen der ringförmigen Anastomosen an den Organen des Verdauungskanals vorausbestimmt ist, der seinen Schaft (1) besitzt, an dessen einem Ende ein Abstützungskopf (2) zur Formgebung der ringförmigen Anastomose angeordnet ist, dadurch **gekennzeichnet**, daß der Schaft (1) aus zwei Schaftteilen (5 und 6) unterschiedlicher Länge zusammengestellt ist, von denen der Teil (5) des Schaftes (1) mit dem Abstützungskopf (2) eine kleinere Länge als bei dem anderen Schfatteil (6) besitzt und in Bezug auf diesen anderen Schaftteil (6) des Schaftes (1) festgelegt ist.

2. Stift nach Anspruch 1, dadurch **gekennzeichnet**, daß der Teil (5) des Schaftes (1) mit dem Abstützungskopf (2) unter Ermöglichung eines Durchstechens des zusammenzunähenden Organs (16) ausgeführt ist.

3. Stift nach Anspruch 2, dadurch **gekennzeichnet**, daß zur Ermöglichung des Durchstechens des zusammenzunähenden Organs (16) das freie Ende (10) des Teils (5) des Schaftes (1) mit dem Abstützungskopf (2) zugespitzt ausgeführt ist.

4. Stift nach Anspruch 2, dadurch **gekennzeichnet**, daß zur Ermöglichung des Durchstechens des zusammenzunähenden Organs (16) er mit einem abnehmbaren Ansatzstück (11) mit seinem zugespitzten freien Ende (13) bestückt ist, das an dem freien Ende des Teils (5) des Schaftes (1) mit dem Abstützungskopf (2) festgelegt ist.

FIG. 3

FIG. 2

FIG. 1

5

FIG. 6

FIG. 5

FIG. 4

# INTERNATIONAL SEARCH REPORT

International Application No PCT/SU 89/00102

## I. CLASSIFICATION OF SUBJECT MATTER (If several classification symbols apply, indicate all) ⁶

According to International Patent Classification (IPC) or to both National Classification and IPC

Int.Cl.$^5$    A 61 B 17/11

## II. FIELDS SEARCHED

### Minimum Documentation Searched 7

| Classification System | Classification Symbols |
|---|---|
| Int.Cl.$^5$ | A 61 B 17/04, 17/06, 17/08, 17/10, 17/11 |

Documentation Searched other than Minimum Documentation
to the Extent that such Documents are Included in the Fields Searched ⁸

## III. DOCUMENTS CONSIDERED TO BE RELEVANT ⁹

| Category ⁺ | Citation of Document, ¹¹ with indication, where appropriate, of the relevant passages ¹² | Relevant to Claim No. ¹³ |
|---|---|---|
| X | SU, A1, 1242140 (Vsesojuzny nauchno-issledovatelsky i ispytatelny institut meditsinskoi tekhniki), 07 July 1986 (07.07.86) | 1 |
| A | EP, A2, 0202090 (ETHICON INC.) 20 November 1986 (20.11.86) abstract, figure 1 <br><br> & US 4671280 <br> JP 61293445 | 2-3 |
| A | SU, A1, 906541 ( N.N.Kanshin et al.) 23 February 1982 (23.02.82) claims (cited in the application) | 3 |
| A | US, A, 4345, 601 (Mamoru Fukuda), 24 August 1982 (24.08.82), abstract | 4 |

--------

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 17 November 1989 (17.11.89) | 08 January 1990 (08.01.90) |
| International Searching Authority <br><br> ISA/SU | Signature of Authorized Officer |

Form PCT/ISA/210 (second sheet) (January 1985)